Europäisches Patentamt

European Patent Office

Office européen des brevets

(19)

(11) Publication number: **0 338 790**
**A2**

# (12) EUROPEAN PATENT APPLICATION

(21) Application number: **89303857.0**

(22) Date of filing: **19.04.89**

(51) Int. Cl.⁴: **C 12 P 13/08**
C 12 P 13/12, C 12 N 15/00,
C 12 N 1/16, C 12 N 1/20,
C 12 P 13/06

(30) Priority: **20.04.88 US 183912**

(43) Date of publication of application:
**25.10.89 Bulletin 89/43**

(84) Designated Contracting States:
**AT BE CH DE ES FR GB GR IT LI LU NL SE**

(71) Applicant: **BIOTECHNICA INTERNATIONAL, INC.**
**85 Bolton Street**
**Cambridge Massachusetts 02140 (US)**

(72) Inventor: **Backman, Keith C.**
**200 Carlisle Road**
**Bedford Massachusetts 01730 (US)**

(74) Representative: **Deans, Michael John Percy et al**
**Lloyd Wise, Tregear & CO. Norman House 105-109 Strand**
**London WC2R OAE (GB)**

(54) **Production of homoserine and its derivatives such as threonine derivatives and methionine precursors.**

(57) The methionine salvage pathway which is normally used to produce polyamines using ATP as a carbon source, can provide significant amounts of homoserine for use in biosynthesis of threonine, threonine derivatives and methionine precursors. Specifically, a cell is engineered to produce S-adenosylmethionine hydrolase, thereby providing a synthetic route to the above compounds which avoids feedback regulation upstream of homoserine, and which relies on a carbon source (ATP) that is replenished as a priority.

FIG 2

EP 0 338 790 A2

Bundesdruckerei Berlin

## Description

## PRODUCTION OF HOMOSERINE AND ITS DERIVATIVES SUCH AS THREONINE DERIVATIVES AND METHIONINE PRECURSORS

This invention relates to methods for producing homoserine and its derivatives, particularly threonine, threonine derivatives, and methionine precursors; it also relates to genetically engineered cells used in the method.

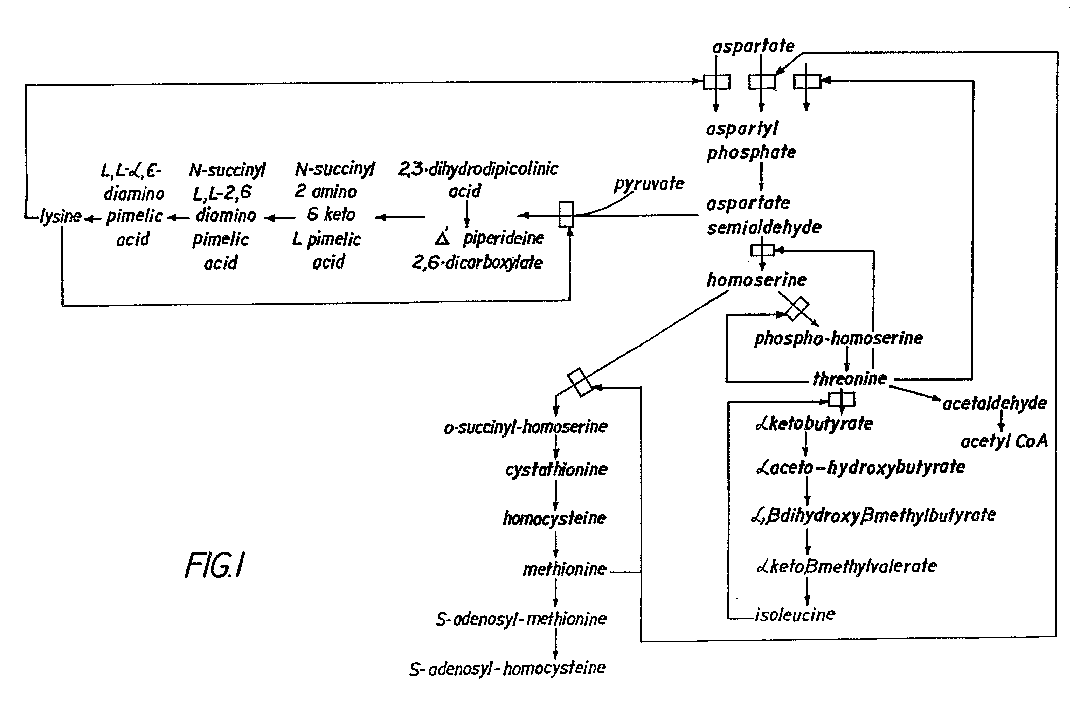

Homoserine is an intermediate in the microbial biosynthetic pathway that is the predominant source of threonine, isoleucine, and methionine for these organisms. Those biosynthetic pathways are shown in Figure 1.

Threonine, isoleucine, and methionine are "essential" amino acids for most vertebrates; i.e., in general, they cannot be metabolically synthesized by vertebrates. These amino acids therefore are useful as additives for both human and animal foods.

As illustrated in Fig. 1, the conventional microbial synthesis pathway is a multi-step process by which aspartate is first converted to homoserine by three enzymatic steps, and homoserine is then converted to other amino acids, e.g., threonine, through additional enzymatic steps. The conventional microbial threonine synthesis pathway, as well as the synthesis pathway for other amino acids, are subject to various types of feedback control, both from reduced production of the enzymes responsible for the synthesis pathway conversions and from feedback inhibition of the operation of some of those enzymes. In this way, microbial cells avoid squandering carbon and energy in the production of these amino acids beyond their needs.

In order to produce significant recoverable quantities of these amino acids, it is useful to circumvent these feedback mechanisms. Prior attempts to produce threonine by channeling the carbon flow into the synthesis of aspartate have involved alteration of the genes responsible for threonine biosynthesis to avoid feedback controls. Techniques for avoiding feedback control include classical mutagenesis (UV, chemical mutagens, ionizing radiation) or recombinant DNA techniques. Specifically, U.S. Patents 4,278,765 and 4,321,325 disclose genetically engineering a bacterial cell to contain multiple copies of the threonine synthesis genes, thrA, thrB, and thrC, that have been altered so as to exhibit reduced sensitivity to regulation by the end-product, threonine. Other techniques include amplifying the threonine biosynthetic gene dosage using a multi-copy plasmid.

In order to maintain the stability of strains which have been mutated or engineered to overproduce threonine, it is known to include on the plasmid an independent genetic element containing a selectable genetic marker that obviates the need for antibiotic selection, for example, a gene which bears a mutation which obviates streptomycin dependence. See, U.S. Patent 4,371,615.

We have discovered that S-adenosylmethionine, supplied in a pathway described below which we will call the methionine salvage pathway, can be a particularly good carbon source for producing recoverable quantities of threonine, threonine derivatives, methionine precursors, homoserine, and other homoserine derivatives. The methods described herein are particularly useful for producing these various compounds in recoverable quantities from S-adenosylmethionine in relatively few steps, because they avoid the need to deregulate multiple steps upstream of homoserine in conventional synthesis pathways, while taking advantage of natural homeostatic systems to replace the carbon source being used.

As described in more detail below, carbon is supplied to the methionine salvage pathway by the ribose unit of ATP, which the cell replenishes as a priority. More specifically, the methionine salvage pathway uses methionine in a quasi catalytic fashion to convert ATP and putrescine to polyamines (spermine and spermidine). One component in that pathway is S-adenosylmethionine, and the invention features tapping into the pathway by means of S-adenosylmethionine hydrolase, to produce homoserine and its derivatives including threonine, $\alpha$-ketobutyric acid, $\alpha$-aceto-$\alpha$-hydroxybutyric acid, $\alpha$, $\beta$-dihydroxy-$\beta$-methylbutyric acid, $\alpha$-keto-$\beta$-methylvaleric acid, and isoleucine. Other homoserine derivatives that can be produced include methionine precursors such as O-succinylhomoserine, cystathione, and homocysteine.

Accordingly, one aspect of the invention features, in general, making a product selected from threonine, threonine derivatives, and methionine precursors, using a cell that is able to carry out the methionine salvage pathway and is capable of expressing S-adenosylmethionine hydrolase ("SAMase"). Preferably a cell capable of carrying out the methionine salvage pathway is genetically engineered to express SAMase, e.g. by including DNA encoding and expressing SAMase. Genetic engineering means adding, deleting or intentionally modifying a known genetic element in a known way, as distinguished from chemical mutagenesis which involves random genetic changes followed by selection of desired phenotypes. These cells are grown for a time and under conditions to convert S-adenosylmethionine to homoserine, concurrently with the operation of the methionine salvage cycle, and a significant yield of the product is recovered.

In preferred embodiments, the method includes recovering a product selected from threonine, and threonine derivatives, including $\alpha$-ketobutyric acid, $\alpha$-aceto-$\alpha$ hydroxybutyric acid, $\alpha,\beta$-dihydroxy-$\beta$-methylbutyric acid, $\alpha$-keto $\beta$-methylvaleric acid, and isoleucine. Alternatively, the method can be used to recover methionine precursors: O-succinylhomoserine, cystathionine, and homocysteine. Where the product is threonine or a threonine derivative, it is preferable to modify the host cell to enhance conversion of homoserine to threonine (particularly, to enhance conversion of homoserine to phosphohomoserine) in the presence of threonine. Such enhancement can be achieved by over-expressing the relevant enzyme (e.g. thrB or thrC), e.g. by including DNA encoding the enzyme on a multi-copy plasmid. Conversion may also be enhanced by cloning a gene encoding an enzyme (e.g. a thrB mutant gene) that exhibits reduced feedback inhibition to the end product. By over-express, we mean expressing effective quantities of enzyme in the presence of levels of threonine which limit expression in wild type organisms. Where the product is threonine, it is also advantageous to further modify the host cell to reduce or eliminate expression of effective levels of enzymes that convert or alter threonine to other substances. For example, the cell can be modified to reduce or eliminate effective amounts of the following enzymes: threonine aldolase, threonine dehydratase, or threonine dehydrogenase. It is also useful in producing threonine and threonine derivatives to express enzymes which exhibit reduced inhibition in response to threonine, specifically the products of the thrB and thrC genes.

Preferably, the DNA for expressing S-adenosylmethionine hydrolase is positioned for transcription from a regulated promoter such as the lacuv5, tac, or trp promoter. SAMase encoding DNA can be derived from the bacteriophage T3 genome and it can be included on the same vector as the DNA encoding thrB and/or thrC gene products. "Derived from T3" means having a nucleic acid sequence that encodes the T3 gene product or a gene product having conservative amino acid substitutions and retaining the desired function. The host cell can be a microorganism, such as a yeast or bacterium; alternatively, the method can include growing in tissue culture a mammalian cell that has been genetically engineered to express the threonine biosynthetic pathway and to express S-adenosylmethionine hydrolase.

In a second aspect, the invention features, generally, a cell which has been genetically engineered to express S-adenosylmethionine hydrolase and to overproduce a product selected from threonine, threonine derivatives, and methionine precursors. Modifying a cell to overproduce a product means avoiding feedback regulation in the product's synthesis pathway, e.g., feedback inhibition of the enzyme, or feedback regulation of synthesis of the enzyme, to generally produce higher levels of the product than a wild-type organism. A modification to overproduce an enzyme is one that reduces or avoids wild-type feedback regulation of the production of the enzyme.

Preferred embodiments of the cell include the modifications described above concerning the preferred embodiments of the method.

In a third aspect, the invention features cells and methods for making homoserine or phospho-homoserine by engineering the cells to include the SAMase gene and to render them incapable of converting homoserine to threonine. A significant yield (more than is recoverable from cells which have not been altered to prevent the homoserine → threonine conversion) is recovered.

Other features and advantages of the invention will be apparent from the following description of the preferred embodiment thereof and from the claims. The drawings will first be described.

Fig. 1 shows the conventional biosynthetic pathway leading from aspartate, via homoserine, to threonine, isoleucine, and methionine. The points of feedback inhibition by the amino acid end products are indicated with solid arrows and open boxes.

Fig. 2 shows the methionine salvage cycle linked to the threonine and methionine biosynthetic pathways through the conversion of S-adenosylmethionine to homoserine through the enzyme S-adenosylmethionine hydrolase.

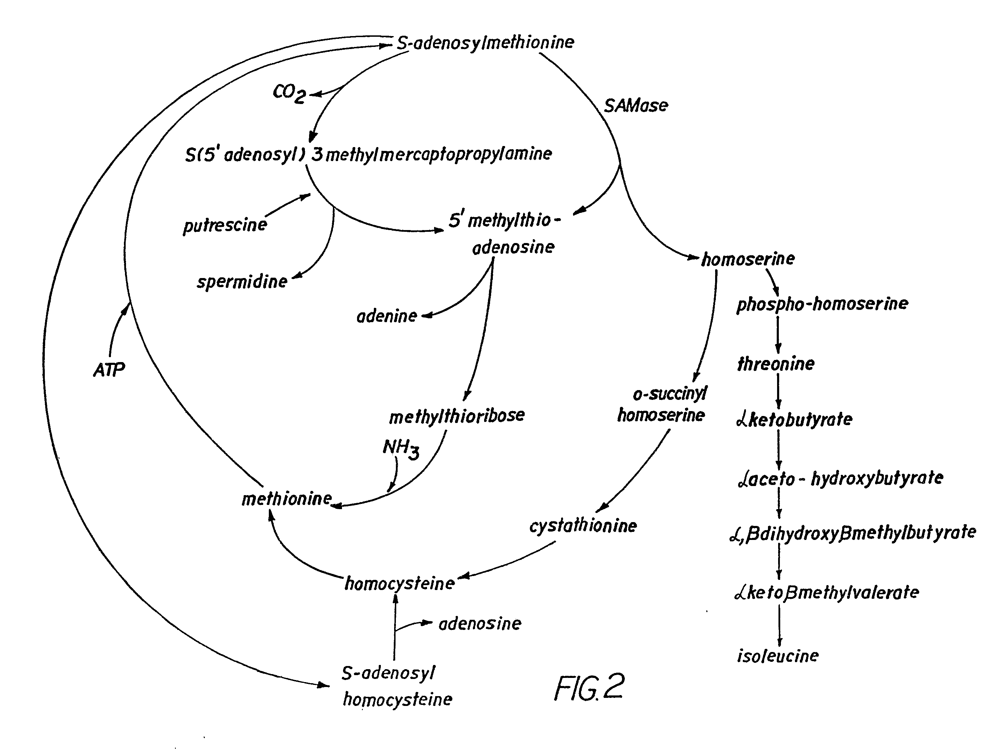

The method is preferably carried out as follows. First, a host organism is selected which does not normally synthesize S-adenosylmethionine hydrolase, but which is capable of carrying out the methionine salvage cycle. Preferred host organisms should provide relatively high levels of S-adenosylmethionine via the methionine salvage pathway. That pathway is illustrated in Fig. 2, and it has been disclosed in various publications, including Marchetto et al, J. Gen. Micro. (1985) 131:2153; Shapiro et al., Biochem. Biophys. Res. Comm. (1981) 102:302; Shapiro et al. Biochem Biophys. Acta (1980) 633:176; and Backlund et al., J. Biol. Chem. (1981) 256:1533. Wang et al. (1982) Plant Physio. 70:117; Yung et al. (1982) Biochem. Biophys. Res. Comm. 104:721.

In that cycle, methionine is used in a quasi catalytic fashion (i.e., there is no net loss of methionine), and ATP and putrescine are consumed to generate polyamines (e.g., spermine and spermidine) which serve to stabilize membrane structures and nucleic acid of bacteria. See Lehninger, Biochemistry 2d Ed. 1975 at p. 718. Specifically, in the methionine salvage pathway, ATP and methionine are joined to form S-adenosylmethionine, which is then decarboxylated. The resulting product, in combination with putrescine, yields spermidine and 5' -methylthioadenosine. To salvage the methionine and complete the cycle, 5'-methylthioadenosine is converted to methionine by several steps that consume an organic phosphate and a source of nitrogen, while generating adenine and a $C_1$ compound.

The primary carbon source consumed is ATP, and the altered cycle will be operative so long as the cell has sufficient ATP available. For most cells, ATP will be replaced as a high priority because it is the primary energy storage medium for the cell. Preferred host cells are those which naturally possess an effective methionine salvage cycle as generally outlined above, and which are capable of effective ATP replacement. While the natural methionine salvage pathway hosts are currently preferred, the invention extends to cells which may be genetically engineered to express that pathway.

Specific useful host organisms that express the methionine cycle include, but are not limited to: yeast, such as Saccharomyces cereviseae, Candida albicans or Candida utilis; mammalian cells in tissue culture (e.g. rat liver cells); and plant cells such as Malus sylvestris (apple) and Lycopersicon esculentum (tomato). Preferred bacterial hosts can be readily identified by those skilled in the field from a collection of isolates using the techniques generally described by Shapiro et al. (1981), cited above. Specifically congeners of the isolate identified by Shapiro et al. (ATCC 8724) are useful. Note that the isolate was originally classified as Enterobacter aerogenes and then later reclassified as Klebsiella oxytoca, and that it therefore apparently has characterisitics of both species. Accordingly, in specifying congeners of the isolate we do not mean to be limited by taxonomic nomenclature and we intend to include organisms that share the general characteristics of ATCC 8724, sufficient for classification therewith. It should be noted that the invention is not limited to the species enumerated above.

Our intention as to the meaning of the term "methionine salvage cycle" is clear from the above description of the cycle; it is not essential that the host cell have the ability to convert S-adenosylmethionine into polyamines and 5'-methylthioadenosine, so long as the remaining elements in the pathway are present, and the cell has available sufficient polyamines to function as a host cell Thus, we do not intend to limit our definition of the host cell to those which are capable of converting S-adenosylmethionine to methylthioadenosine. Other steps in the pathway are illustrated in Fig. 2.

The host organism is genetically engineered to produce S-adenosylmethionine hydrolase ("SAMase", "Ado-Metase", EC 3.3.1.2). The gene encoding SAMase may be obtained from any one of a number of sources, including E. coli bacteriophage T3. See, e.g. Gold et al. (1964) Proc. Nat'l. Acad. Sci. 52:292-297. Specifically, the SAMase gene has been cloned by Hughes et al. J. Bact. 169:3625 3632 (1987), and the technique described therein can be used to obtain the SAMase gene from publicly available starting materials (E. coli bacteriophage T3, ATCC 11303-33). The particular strategy used by Hughes et al. involves cloning the coliphage T3 gene into the M13mp8 cloning vector (Bethesda Research Laboratories, Inc., Bethesda, MD). Other cloning strategies will be apparent to those skilled in the art and the invention is not limited to the strategy disclosed by Hughes et al.

A vehicle to engineer the host organism to produce SAMase can be constructed by placing the SAMase gene under the control of a suitable promoter. It may be useful to regulate expression of the SAMase gene, and thereby requlate the overproduction of the product. A variety of promoters for requlated expression of a heterologous gene are known, including, for example, the lacuv5 E. coli promoter (see, e.g. Maniatis et al., Molecular Cloning, A Laboratory Manual, Cold Spring Harbor 1982). Standard techniques are used to splice the promoter of choice in position to transcribe the SAMase gene.

By way of example, the SAMase gene of T3 can be cloned from bacteriophage T3 DNA, independent of the upstream host specific promoter by isolating the HaeIII fragment extending from position 870 to position 1431 on the T3 genomic map. Hughes et al. (1987) J. Bacteriol 169:3625. The specific T3 sequence is also reported by Hughes et al. Nucl. Acid Res., 15:717-729 (1987).

The SAMase gene can be cloned into the vector pKK223-3 (obtainable from Pharmacia Fine Chemicals) downstream of the tac promoter (a hybrid of the trp -35 region fused to the lac -10 region and the lac operator; DeBoer et al. U.S. Patent No. 4,551,433) as reported by Hughes et al. (N.A.R., 1987, 15:717). Other regulated promoters such as the E. coli lacuv5 or trp promoters, or others which are utilized in Enterobacter, can be cloned upstream of the SAMase gene to direct the expression of the enzyme. The lacuv5 promoter is available for example on pOP203 3 (ATCC No. 37207) or pOP203-13 (ATCC No. 37208). The trp promoter is available for example in E. coli strain W3110 carrying plasmid pSC101-trp.115 (ATCC No. 31743).

For production in yeast, inducible promoters are preferred for expression of the SAMase gene. The GAL1, GAL7 and GAL10 promoters are all available on the lambda clone Sc481 (St. John et al., 1981, J. Mol. Biol., 152:285-315) and the ADR2 promoter is available on the plasmid pADR2-BSa (Williamson et al., 1980, Cell 23:605-614).

For production in mammalian cells, examples of suitable promoters include the SV40 early promoter available on pSV2-cat (Gorman et al., 1982, Mol. Cell Biol., 2:1044; ATCC #37155), the Adenovirus promoter available on pAV1 and pAV2 (Laughlin et al., 1983, Gene 23:65; ATCC #37215 and 37216) and the ROUS Sarcoma virus promoter on the long terminal repeat, available on pRSVcat (Gorman et al., 1982, P.N.A.S. 79:6777; ATCC #37152). Other promoters for yeast and mammalian cell expression systems are available and are well known by one skilled in the art.

Preferably, the host cell is further engineered to enhance recovery of the desired product. For example, for production of threonine and threonine derivatives, the threonine operon genes thrB and thrC, as well as a promoter for the expression of thrB and the thrC, may be included on the same vector that carries the SAMase gene, or on a different vector. A suitable vector can be assembled using techniques such as those described in Maniatis et al, Molecular Cloning, A Laboratory Manual, Cold Spring Harbor 1982. The vector is then transformed, using conventional techniques, into the desired host strains.

The vector may be an extrachromosomally maintained plasmid or it may contain sequences which allow integration into the chromosome of the host organism. When extrachromosomally maintained, the vector includes a selectable marker, origin of replication, and regulatory DNA (a promoter) that is functional in the host of interest.

The promoter gene fusions may be cloned into a lysogenic bacteriophage, for example, bacteriophage lambda, for integration into a susceptible bacterial host chromosome. Alternatively, vectors (such as those described in EP 0174096. the disclosure of which is hereby incorporated by reference) containing elements sufficient for integrating DNA into a host chromosome can be applied herein for the integration of the genes of interest such as genes for the threonine metabolic enzymes and for SAMase.

For threonine production, the thrB and thrC genes, can be expressed from the same, or a different promoter sequence as the SAMase gene and these genes may be carried on the same or on a separate vector from the SAMase gene. The threonine operon can be isolated from E.coli DNA on a HindIII fragment as described by Debabov et al. (supra.). This fragment can be further digested with a suitable restriction endonuclease to provide a fragment which contains only the thrB and thrC genes. For example, a suitable fragment can be derived from the E.coli threonine operon by cleavage with SnaBI (just upstream of thrB) and BspMII (just downstream of thrC). Other methods of generating fragments, using standard genetic engineering techniques will be apparent to those skilled in the art from an analysis of the published sequence of the thr operon (GENBANK, Accession Number J01706).

Conditions for culturing cells

Cells are cultured under optimum conditions to promote the repeated cycling of the methionine metabolic pathway. Specifically, the culture conditions should provide carbon and nitrogen sources which stimulate the replacement of S-adenosylmethionine from the methionine salvage cycle. In the methionine salvage cycle, ATP and fixed ammonia are consumed. In order to force the cycle towards product formation, ATP and ammonia must be resupplied. ATP can be replenished through the glycolytic cycle if a continuous supply of carbohydrate or saccharide, such as glucose, is provided. Fixed ammonia can be made available through a source of nitrogen, nitrate or ammonia.

The cell is also preferably modified to reduce conversion of the desired product to other products. For example, conversion of threonine to isoleucine is blocked by deletion of the enzyme (threonine dehydratase) required to convert threonine to α-ketobutyrate. See, e.g.. U.S. Pat. 4,278,765, the disclosure of which is hereby incorporated by reference.

Cell culturing techniques are used which are appropriate for the chosen host strain. Media formulations for the culturing of bacteria, fungi and cell lines are available in the ATCC Media Handbook, 1984, Library of Congress Catalog Card Number: 84-71497. General methods for plant cell culture techniques are available in the Handbook of Plant Cell Culture (1983, eds. D.A. Evans, W.R. Sharp, P.V. Ammirato and Y. Yamada, Macmillan Publishing Co., N.Y., N.Y.).

Following fermentation, the desired product is recovered by conventional methods.

Other embodiments are feasible,

The method may be used to produce other substances by appropriate modifications to the host organism. For example, to produce isoleucine, feedback inhibition of isoleucine synthesis is avoided by obtaining feedback resistant mutant host strains (particularly strains overproducing feedback deregulated threonine dehydratase) using isoleucine analogs according to the general technique of U.S. Pat. 4,329,427, and engineering those host strains as described above to produce homoserine from S-adenosylmethionine.

Intermediates between homoserine and isoleucine can be produced by interrupting the known synthesis pathway shown in Fig. 1 at the desired point. For example, the gene responsible for the step downstream from the desired product can be deleted or mutated to interrupt the isoleucine synthesis pathway.

The method can also be used to synthesize precursors to methionine in the conventional methionine synthesis pathway (Fig. 1), by interrupting that pathway at the desired point, e.g. by deleting or mutating the gene producing the enzyme responsible for that pathway step.

Those skilled in the art are aware of techniques such as complementation of deficient mutants for identifying and cloning the genes to be mutated as described above.

## Claims

1. A method of making a product selected from threonine, threonine derivatives, and methionine precursors, the method being characterised in comprising the steps of:
    a) providing a cell capable of carrying out the methionine salvage pathway and capable of expressing S-adenosylmethionine hydrolase;
    b) growing said cell for a time and under conditions to convert S-adenosylmethionine to homoserine and to convert homoserine to said product; and
    c) recovering said product.

2. A method according to Claim 1, wherein said product is threonine, or a threonine derivative, the method being further characterised in that said cell has been modified from a wild type cell to enhance the wild-type levels of conversion of homoserine to threonine in the presence of threonine.

3. A method according to Claim 2, further characterised in that said cell has been modified to enhance wild-type levels of conversion of homoserine to phospho-homoserine in the presence of threonine.

4. A method according to Claims 3 or 4, characterised in that said cell has been further modified to reduce or eliminate expression of effective amounts of at least one enzyme that alters threonine, preferably at least one of the following enzymes: threonine aldolase, threonine dehydratase, or threonine dehydrogenase.

5. A method according to Claim 1, further characterised in that said product is a methionine precursor selected from o-succinyl homoserine, cystathionine, and homocysteine.

6. A method according to any preceding claim, further characterised in that said cell has been genetically engineered to produce SAMase.

7. A method according to Claim 6, further characterised in that said cell has been genetically engineered by inclusion of DNA encoding S-adenosylmethionine hydrolase, said DNA being positioned for transcription from a regulated promoter.

8. A method according to Claim 6, further characterised in that said cell has been modified by inclusion of DNA encoding SAMase derived from the bacteriophage T3 genome.

9. A method according to Claim 6, further characterised in that said cell includes DNA capable of expressing SAMase on an extrachromosomal vector.

10. A method according to Claim 9, further characterised in that said cell includes on said vector thrB and thrC genes.

11. A method according to any preceding claim, further characterised in that said cell is a microorganism.

12. A method according to Claim 11, further characterised in that said microorganism is a bacterium or yeast, preferably a yeast selected from Saccharomyces cereviseae, Candida albicans or Candida utilis.

13. A method according to any of Claims 1 to 10, further characterised in that said cell is a mammalian cell genetically engineered to express the threonine biosynthetic pathway and in that said method comprises growing said cell in tissue culture.

14. A cell that is capable of carrying out the methionine salvage pathway and capable of expressing S-adenosylmethionine hydrolase, said cell being modified to over-produce a product selected from threonine, threonine derivatives, and methionine precursors.

15. A cell according to Claim 14, further characterised in that it has been genetically engineered to produce SAMase.

16. A cell according to Claim 14, further characterised in that said cell has been modified from a wild-type cell to enhance wild-type levels of conversion of homoserine to threonine in the presence of threonine.

17. A cell according to Claim 16, further characterised in that said cell has been modified to enhance wild-type levels of conversion of homoserine to phospho-homoserine.

18. A cell according to Claim 16, further characterised in that it has been further modified to reduce or eliminate expression of effective amounts of at least one enzyme that alters threonine, preferably at least one of the following enzymes: threonine aldolase, threonine dehydratase or threonine dehydrogenase.

19. A cell according to Claim 18, further characterised in that it carries on the same vector, thrB and thrC genes and a gene encoding S-adenosylmethionine hydrolase.

20. A cell according to Claim 15, characterised in that it has been genetically engineered by inclusion of DNA encoding SAMase, said DNA being positioned for transcription from a regulated promoter.

21. A cell according to Claims 14 or 15, further characterised in that said cell comprises DNA encoding SAMase derived from the T3 bacteriophage genome.

22. A cell according to Claim 21, further characterised in that said DNA is positioned on an extrachromosomal vector.

23. A cell according to any of Claims 14 to 22, further characterised in that said cell is a microorganism.

24. A cell according to Claim 23, further characterised in that said microorganism is a bacterium or yeast, preferably a yeast selected from Saccharomyces cereviseae, Candida albicans or Candida utilis.

25. A method of producing homoserine or homoserine phosphoric acid, characterised in comprising the steps of:

(a) providing a cell capable of carrying out the methionine cycle, which has been modified by inclusion of DNA capable of expressing S-adenosylmethionine hydrolase, said cell being substantially incapable of converting homoserine to threonine;

(b) growing said cell for a time and under conditions to convert S-adenosylmethionine to homoserine; and

(c) recovering a significant yield of homoserine or phosphohomoserine.

26. A cell capable of carrying out the methionine cycle, characterised in that said cell has been genetically engineered to express a gene encoding S-adenosylmethionine hydrolase, said cell being substantially incapable of converting homoserine to threonine.

FIG.I

EP 0 338 790 A2

FIG.2

EP 0 338 790 A2